# EUROPÄISCHE PATENTANMELDUNG

(11) **EP 0 872 737 A1**
(43) Veröffentlichungstag der Anmeldung: **21.10.1998**
(21) Anmeldenummer: 97106135.3
(22) Anmeldetag: 15.04.1997
(51) Int. Cl.: G01N 33/94

(54) **Ein objektivierbares Verfahren zur Feststellung einer Überforderung oder Unterforderung in Belastungssituationen**

(71) Anmelder: Zimmermann, Elke, Prof. Dr., 33619 Bielefeld (DE)
(72) Erfinder: Zimmermann, Elke, Prof.-Dr., 33619 Bielefeld (DE); Liebel, Franz-Peter, Dr., 33619 Bielefeld (DE)

(57) **Zusammenfassung**

Das Verfahren bietet die Möglichkeit, durch objektive Messungen eine Unterforderung oder Überforderung von Personen in konkreten Belastungs- oder Arbeitssituationen zu ermitteln.
Hierzu werden die Konzentrationen der Botenstoffe des vegetativen Nervensystems Noradrenalin (NA) und Adrenalin (A) aus Urinproben bestimmt. Die so gewonnenen NA/A-Quotienten erlauben die angestrebten Aussagen, wobei tabellarische Auflistungen verwendet werden, die eine Zuordnung der NA/A-Quotienten zur jeweiligen Qualität der Arbeitsausführung enthalten.

## Beschreibung

### 1. Verwendbarkeit der Erfindung

Die Erfindung ist dann anzuwenden, wenn ein Instrument zur objektivierbaren Beurteilung der Unterforderung oder Überforderung eines tätigen Menschen benötigt wird. Die Erfindung ist insbesondere dann einzusetzen, wenn die erbrachte Leistung nicht unmittelbar gewertet werden kann, wie zum Beispiel bei Linienpiloten, wenn aber eine Überforderung der tätigen Person von erheblichem Nachteil wäre.

Die Erfindung ist also anzuwenden, wenn es gilt, die Arbeitskraft des Individuums oder ganzer Arbeitsteams zu sichern. Dazu bedarf es einer objektivierbaren und wissenschaftlich fundierten Methode, die schnell und problemlos die benötigten Informationen bereitstellen kann.

Die Erfindung ermöglicht somit,
- die Eignung von Personen für konkrete Tätigkeiten zu messen,
- den Nutzen eventueller Umstellungen am Arbeitsplatz durch erneute Anwendung des Verfahrens zu ermitteln und
- gegebenenfalls Störgrößen wie Zeitdruck oder Konkurrenzdruck aufzudecken und entsprechend abzustellen.

### 2. Stand der Technik und Vorteile des Verfahrens

Objektive Meßverfahren zur Beurteilung der Überforderung oder Unterforderung von Personen unmittelbar am Tätigkeitsort und bei der ausgeübten Tätigkeit existieren bisher nicht. Dies gilt vor allem für Tätigkeiten, bei denen die erbrachte Leistung nicht unmittelbar erfaßt, bewertet oder gar abgezählt werden kann.

Folgende Eigenschaften der Erfindung gewährleisten einen unmittelbaren Nutzen für alle Beteiligten:
- Die Messung erfolgt ohne Beeinträchtigung der Tätigkeit und damit ohne verfälschende Einflußnahme unter den spezifischen Belastungsbedingungen des zu Untersuchenden.
- Die Messungen können beliebig oft wiederholt werden, um Auswirkungen gezielter Änderungen im Arbeitsprozeß oder im Verhalten des Untersuchten zu erfassen.
- Die Meßergebnisse können anonymisiert als Einzelwerte oder Gruppenwerte die Diskussionsgrundlage für wünschenswerte Änderungen darstellen und dabei die entscheidenden Hinweise für die Art der Änderung liefern.
- Die Meßergebnisse liefern dem Einzelnen unmittelbar nutzbare Informationen zur Verbesserung seiner von Unter- oder Überforderung geprägten Situation.
- Die Erfindung ermöglicht es, daß trotz individueller Unterschiede der untersuchten Personen hinsichtlich Geschlecht, Alter und Erfahrung anhand der Wertetabellen eine Wertung vorgenommen werden kann.
- Die Erfindung ermöglicht die Überprüfung der Effizienz getroffener Maßnahmen im Hinblick auf die Schaffung besserer Voraussetzungen für angestrebte Leistungen.
- Die Erfindung eignet sich zur Schulung des Individuums, indem die Meßwerte als objektive Rückmeldung die subjektive Einschätzung der Leistung präzisieren helfen.

### 3. Darstellung der Lösung

Von entscheidender Wichtigkeit sind die in Anspruch 1 enthaltenen Wertetabellen, die eine Zuordnung der NA/A-Quotienten zu den Aussagen über die Qualität der Arbeitsausführung ermöglichen. Diese Tabellen wurden wie folgt ermittelt:
- Leistungssportler aus Sportarten mit zuverlässig meßbarer Leistungsdokumentation wurden unter Trainings- und Wettkampfbedingungen dem in Anspruch 1 beschriebenem Verfahren zur Bestimmung der NA/A-Quotienten unterzogen.
- Der Zusammenhang zwischen erbrachter Leistung und NA/A-Quotienten zeigte unabhängig von der Sportart, daß die besten Leistungen - und das war bei den untersuchten Sportlern das Niveau von Weltrekordhaltern und Olympiasiegern - in einem eng umschriebenen NA/A-Quotientenbereich von 2.8 bis 5 erbracht wurden.
- Es zeigte sich des weiteren, daß dieses Leistungsniveau nur stabilisiert werden konnte, wenn der genannte NA/A-Quotientenbereich nicht nur im Wettkampf, sondern auch im Training beibehalten wurde. Abweichungen von diesem Bereich führen im Wettkampf zu Leistungseinbußen, im Training führen sie zu sportartspezifischen Überlastungsreaktionen trotz geringerer Anforderung bei den Einzelbelastungen.
- Die gleichen Zusammenhänge zeigten sich in Sportarten, in denen neben den physischen auch die kognitiven Anforderungen über das Leistungsniveau entscheiden.
- Anhand dieser Beobachtungen konnte das Zusammenspiel zwischen Zentralnervensystem (ZNS) und Skelettmuskulatur in leistungsfordernden Situationen verdeutlicht werden. Mit den Erkenntnissen der Physiologie über die Wirkungen von Noradrenalin und Adrenalin auf die Skelettmuskulatur waren die Voraussetzungen gegeben, um die Bedeutung des sympathischen Nervensystems für physische Belastungen zu erklären, insbesondere wenn hohe Anforderungen an die muskulärer Koordination, Reaktionsfähigkeit und Präzision der Entscheidung bestehen.
- Unabhängig von den individuellen Besonderheiten einer Person (Alter, Geschlecht, Erfahrung, Gesundheit) liefert der NA/A-Quotient als Maß für die Sympathikusaktivität präzise Auskunft darüber, inwieweit die Skelettmuskulatur auf Unterstützung seitens des ZNS rechnen darf. Damit steht ein einfach handhabbares System für die Beurteilung der Überforderung und Unterforderung in physischen Belastungssituationen zur Verfügung.
- Das Verfahren wurde unter Nutzung der Erfahrung im Leistungssport an Personen in unterschiedlichen Tätigkeitsbereichen der Ausbildung und des Berufslebens (Schüler, Studenten, Trainer, Lehrer, Ärzte, Piloten, Musiker, Wissenschaftler) erprobt. Die subjektive Einschätzung der Belastung und Benennung von Gründen für erkannte Leistungseinbußen war den Betroffenen allenfalls in Form pauschaler Hinweise auf Streß möglich, erst unter Zuhilfenahme der NA/A-Quotienten konnten hilfreiche Maßnahmen vorgeschlagen und erfolgreich umgesetzt werden.
- Die Untersuchung an Personen in Belastungs- und Arbeitssituationen zeigte, daß die Bedeutung der Erholungsphase deutlich unterschätzt wird. Wie bereits im Leistungssport erkannt, konnte anhand der Meßergebnisse ein Empfinden für die in der aktiven Belastungsphase und die in der Erholungsphase jeweils geeignete Sympathikusaktivität entwickelt werden. Nach 5 bis 7 Messungen gelingt interessierten Personen die korrekte Einschätzung, und sie können unabhängig von weiteren Messungen geeignete Maßnahmen zur Einflußnahme auf die Sympathikusaktivität im Sinne einer Vermeidung von Überforderung und Unterforderung einleiten.

Als praktikables Verfahren zur Probennahme und NA/A-Bestimmung empfiehlt sich folgende Vorgehensweise:
- Der zu Beurteilende sammelt während der interessierenden Tätigkeit jede anfallende Urinprobe in einem dafür vorgesehenen verschließbaren Gefäß (Fassungsvermögen ca. 10ml). Es hat sich bewährt, ein größeres Gefäße mit einem Fassungsvermögen von 500ml und leicht ablesbarer Skalierung für das Auffangen der Probe bereitzuhalten. Für das Labor füllt der Proband eine kleine Menge aus dem großen in das kleine Gefäß.
   Ist die Tätigkeit von vergleichsweise kurzer Dauer, sollte unmittelbar vor Aufnahme und sobald wie möglich nach Beendigung der Tätigkeit jeweils eine Probe gewonnen werden, um andere Einflußfaktoren weitestgehend auszuschließen.
   Während für klinische Fragestellungen besondere Hinweise zur Ernährung, Medikamenteneinnahme und zu anderen möglichen Einflüssen auf die Noradrenalin- und Adrenalinkonzentration zu beachten sind, ist es für das hier eingesetzte Verfahren von Bedeutung, daß die zu untersuchenden Personen sich so verhalten wie immer. Erst wenn man sich von der Änderung der Ernährung eine wünschenswerte Einflußnahme auf die Sympathikusaktivität verspricht, soll eine vergleichende Untersuchung mit kontrollierter Änderung vorgenommen werden.
- Die Proben werden codiert oder in Klartext beschriftet, und bis zur Messung kühl (oder tiefgekühlt) und dunkel aufbewahrt.
   Neben der Angabe zur Person erfordert die Meßwertinterpretation Angaben zur Belastungs- bzw. Erholungssituation, zur Uhrzeit der Probennahme und zur Zeitspanne der Sammelphase. Für ergänzende Meßwertinterpretationen (zum Beispiel zur Beurteilung der Höhe der physischen Belastung) ist die Angabe zum Gesamtvolumen des in der Sammelphase gebildeten Urins erforderlich (vom Probanden bestimmbar mittels der Skalierung des großen Sammelgefäßes).
- Die Messung der Noradrenalin- und Adrenalinkonzentration erfolgt in einem Meßgang mittels gaschromatographischer Trennung und massenspektrometrischer Detektion oder mittels Hochdruck-Flüssigkeitschromatographischer Trennung und elektrochemischer Detektion.
- Die ermittelten NA/A-Quotienten werden anhand der Wertetabellen interpretiert und zur Veranschaulichung gegebenenfalls in graphischer Form dargestellt.

## Patentansprüche

1. Das Verfahren dient zur Feststellung der Überforderung oder Unterforderung von Personen in Belastungs- und Arbeitssituationen,
dadurch gekennzeichnet, daß
- zunächst von dem zu Beurteilenden während der Zeit seiner Tätigkeit und in Erholungsphasen Urinproben gesammelt werden,
- daß weiter anhand der Proben im Labor die Noradrenalin- und Adrenalin-Konzentrationen (NA- und A-Konzentrationen) bestimmt werden,
- daß schließlich anhand der ermittelten NA/A-Quotienten, und zwar der NA/A-Quotienten für aktive Phasen (NA/A-akt) sowie für Erholungsphasen (NA/A-erh), eine Beurteilung der Leistungsfähigkeit mittels dreier Wertetabellen vorgenommen wird, wobei die 1. Wertetabelle für physisch belastende Tätigkeiten eine Beurteilung beinhaltet gemäß
| | |
|---|---|
| NA/A-akt <2.0 | es besteht eine leistungsmindernde Anspannung, die auch bei gut vorbereiteten Personen zu Fehlentscheidungen und Beeinträchtigungen der Bewegungskoordination führt, |
| NA/A-akt 2.0 - 6.0 bei NA/A-erh <6.0 | es besteht die Gefahr einer Überforderung bei langfristiger Durchführung physisch anspruchsvoller Arbeit, |
| NA/A-akt 2.0 - 6.0 bei NA/A-erh >8.0 | es bestehen die besten Voraussetzungen zur Durchführung physisch und/oder koordinativ anspruchsvoller Arbeit, |
| NA/A-akt 6.1 - 8.0 | es bestehen akzeptable Voraussetzungen zur Durchführung physisch wenig belastender Arbeit mit ruhigem Bewegungsablauf, |
| NA/A-akt >8.0 bei Beanspruchung kleiner Muskelgruppen | es bestehen akzeptable Voraussetzungen zur Durchführung von Arbeiten mit geringfügiger Belastungsintensität und mit stereotypen Bewegungsabläufen, |
| NA/A-akt >8.0 bei Beanspruchung großer Muskelgruppen | es besteht die Gefahr einer Überbeanspruchung des Organismus oder seiner Teilsysteme bei physisch belastender Arbeit, |
| NA/A-akt >10.0 bei NA/A-erh >15.0 | wiederholt auftretende Meßwerte in diesen Bereichen sind mit dem Anspruch auf körperliche Leistung nicht vereinbar und auch Schlafphasen ergeben kaum eine Erholung, |
| und wobei die 2. Wertetabelle für den kreativen Belastungsbereich eine Beurteilung beinhaltet gemäß | |
| NA/A-akt <2.0 | es besteht eine leistungsmindernde Anspannung, die auch bei gut vorbereiteten Personen zu Fehlentscheidungen und Beeinträchtigungen der Bewegungskoordination führt, |
| NA/A-akt 2.0 - 6.0 bei NA/A-erh < 4.0 | es besteht die Gefahr einer Überforderung bei langfristiger Durchführung kreativ anspruchsvoller Arbeit, |
| NA/A-akt 2.0 - 6.0 bei NA/A-erh >8.0 | es bestehen die besten Voraussetzungen zur Durchführung kreativ anspruchsvoller Arbeit, insbesondere wenn diese mit physischer Belastung verbunden ist, |
| NA/A-akt 6.1 - 10.0 | es bestehen gute Voraussetzungen zur Durchführung kreativ fordernder, physisch wenig belastender Arbeit in ruhiger Umgebung, |
| NA/A-akt >10.0 | es bestehen ungünstige Voraussetzungen für kognitiv anspruchsvolles Arbeiten im kreativen Bereich, |
| NA/A-akt >10.0 bei NA/A-erh >15.0 | wiederholt auftretende Meßwerte in diesen Bereichen sind mit dem Anspruch auf kreative Leistung nicht vereinbar und selbst Schlafphasen ergeben kaum eine Erholung, |
| und wobei die 3. Wertetabelle für Tätigkeiten mit monotonen Handlungsabläufen eine Beurteilung beinhaltet gemäß | |
| NA/A-akt <2.0 | es besteht eine leistungsmindernde Anspannung, die auch bei gut vorbereiteten Personen zu Fehlentscheidungen und Beeinträchtigungen der Bewegungskoordination führt, |
| NA/A-akt 2.0 - 6.0 bei NA/A-erh <6.0 | es besteht die Gefahr einer Überforderung bei langfristiger Durchführung physisch anspruchsvoller Arbeit in diesem Bereich, |
| NA/A-akt 2.0 - 6.0 bei NA/A-erh >8.0 | es bestehen die besten Voraussetzungen zur Durchführung physisch anspruchsvoller Arbeit, |
| NA/A-akt 6.1 - 10.0 | es bestehen akzeptable Voraussetzungen zur Durchführung physisch wenig belastender Arbeit mit ruhigem Bewegungsablauf, |
| NA/A-akt >10.0 bei NA/A-erh >15.0 | wiederholt auftretende Meßwerte in diesen Bereichen sind mit dem Anspruch auf körperliche Leistung nicht vereinbar und auch Schlafphasen ergeben kaum eine Erholung. |

2. Verwendung des Verfahrens gemaß Anspruch 1, wenn bei den zur Beurteilung herangezogenen Wertetabellen andere Einteilungen vorgenommen werden, dadurch gekennzeichnet, daß die Bereiche vergrößert und/oder verkleinert und die zugehörigen Aussagen in anderer Formulierung gefaßt werden, oder wenn statt der NA/A-Quotienten die reziproken A/NA-Quotienten verwendet werden, oder wenn die Wertetabellen durch weitere NA/A-erh-Werte ergänzt werden.

3. Vorgehen gemäß Anspruch 1 und 2, wobei nach erfolgter Beurteilung der Leistungsfähigkeit geeignete Maßnahmen zur Verbesserung der Leistungsfähigkeit getroffen werden und das Verfahren gemäß Anspruch 1 und 2 erneut eingesetzt wird, um den Einfluß der getroffenen Maßnahmen festzustellen.

4. Verfahren gemäß Anspruch 1 bis 3, wenn als weitere Einflußgröße Dopamin verwendet wird, dadurch gekennzeichnet, daß im wesentlichen die Aussagen der Ansprüche 1 bis 3 erhalten bleiben, daß sie jedoch durch den zusätzlichen Einsatz der Einflußgröße Dopamin moduliert werden.

5. Verfahren gemäß Anspruch 1 bis 4, wenn als weitere Einflußgrößen die Hormone der Hypophysen-Nebennieren-Achse
- Adrenocorticotropes Hormon (ACTH),
- Cortisol,
- Cortison,
- Corticosteron,
- Aldosteron,
die Hormone der Hypophysen-Schilddrüsen-Achse
- Thyreoidea Stimulierendes Hormon (TSH),
- Trijodthyronin (T3),
- Thyroxin (T4),
die Hormone der Hypophysen-Leber-Achse
- Wachstumshormon (hGH),
- Insulinlike-Growth-Factor 1 (IGF-1),
- Insulinlike-Growth-Factor 2 (IGF-2)
oder Kenngrößen des Immunsystems
- Zahl der Natural-Killer-Zellen (NK-Zellen),
- Zahl der als Helfer-Zellen und der als Suppressor-Zellen bezeichneten Lymphozyten,
- Quotient aus Helfer-Zellen und Suppressor-Zellen
verwendet werden, dadurch gekennzeichnet, daß im wesentlichen die Aussagen der Ansprüche 1 bis 4 erhalten bleiben, daß diese jedoch durch die zusätzliche Verwendung der genannten Einflußgrößen moduliert werden.

6. Verfahren gemäß Anspruch 1 bis 5, wenn die Beurteilung insbesondere für Personen vorgenommen wird,
- die Luft-, Wasser- und Landfahrzeuge führen,
- die Werkzeuge, Maschinen oder Anlagen bedienen,
- die im medizinischen Bereich oder in Heilberufen arbeiten,
- die als bewaffnete oder unbewaffnete Sicherheits- und Ordnungskräfte eingesetzt sind,
- die als Redner, Unterrichtende oder als Mitarbeiter von wissenschaßlichen oder künstlerischen Teams tätig sind,
- die als Mitarbeiter in Gewerbebetrieben an Produktionsprozessen beteiligt sind,
- die als Amateur- oder als Berufssportler eine Sportdisziplin betreiben.

7. Verfahren gemäß Anspruch 1 bis 5, wenn überprüft werden soll, in welcher Weise eine zu beurteilende Person durch konkrete Lebensumstände, zum Beispiel durch Strafvollzug, durch Heimunterbringung, durch Wehrdienst oder durch vergleichbare Lebensumstände belastet wird, dadurch gekennzeichnet, daß für eine solche Verwendung der Erfindung die in Anspruch 1 enthaltenen Wertetabellen in den Formulierungen geeignet angepaßt werden, insbesondere die 2.Wertetabelle für den nicht-physischen Bereich.

8. Verfahren gemäß Anspruch 1 bis 7, wenn im Falle starker körperlicher Beanspruchung zur Beurteilung des Grades der Angepaßtheit an die Belastung und/oder der Bewegungsökonomie die Noradrenalinausscheidungsrate verwendet wird, dadurch gekennzeichnet, daß zu den NA/A-Quotienten auch die jeweiligen Noradrenalin-Ausscheidungsraten bestimmt werden, und daß sich eine Beurteilungsmöglichkeit dadurch ergibt, daß im NA/A-Quotientenbereich von 2 bis 8 für einen konkreten NA/A-Quotienten die zugehörige Noradrenalin-Ausscheidungsrate um so niedriger ist, je angepaßter oder ökonomischer der Bewegungsablauf gestaltet ist.

9. Verfahren gemäß Anspruch 1 bis 8, wenn für Noradrenalin, Adrenalin und weitere Hormone nicht die Urinkonzentrationen bestimmt werden, sondern wenn Messungen der Blutkonzentrationen erfolgen, oder wenn Messungen von jeweils zugehörigen Metaboliten im Urin und/oder im Blut vorgenommen werden.
